# EUROPEAN PATENT APPLICATION

(11) **EP 3 663 303 A1**
(43) Date of publication of application: **10.06.2020**
(21) Application number: 18840872.8
(22) Date of filing: 20.07.2018
(51) Int. Cl.: C07F 7/08, A61L 27/18, C07F 7/10, C08F 20/26, C07B 61/00

(54) **SILOXANE COMPOUND AND PRODUCTION METHOD FOR SAME**

(30) Priority: 01.08.2017 JP 2017149112
(71) Applicant: Shin-Etsu Chemical Co., Ltd., Tokyo 100-0004 (JP)
(72) Inventor: OKAMURA, Kaoru, Annaka-shi Gunma 379-0224 (JP)
(74) Representative: V.O.
(86) International application number: PCT/JP2018/027346
(87) International publication number: WO 2019/026652

(57) **Abstract**

An object of the present invention is to provide a monomer having a polysiloxane structure and capable of providing a polymer having sufficient compatibility and hydrolysis resistance, while having high oxygen permeability useful for an ophthalmic device, and a method for producing the monomer. The present invention provides a compound represented by the formula (1) which has a radical-polymerizable group at one terminal, one to three alkylsiloxy groups having a hydrophilic and bulky group represented by formula (3-A) or formula (3-B), and has a divalent hydrocarbon group L which has 2 to 20 carbon atoms and may contain one or more selected from an ether bond and a urethane bond, a part of the hydrogen atoms bonded to the carbon atoms may be substituted with a hydroxyl group.

## Description

### TECHNICAL FIELD

The present invention relates to a polysiloxane monomer which has excellent wettability and is suitable for preparing a medical device, and a method for preparing the same. Specifically, the present invention relates to a compound which is excellent in compatibility with other polymerizable monomers such as (meth)acrylic monomers and may be (co)polymerized to provide a (co)polymer which is excellent in hydrolysis resistance and suitable for application in medical devices, including ophthalmic devices. The present invention relates further to a method for producing the compound.

### BACKGROUND OF THE INVENTION

Various siloxane compounds are known as monomers for production of ophthalmic devices. For example, TRIS, 3-[tris(trimethylsiloxy)silyl]propyl methacrylate, represented by the following formula 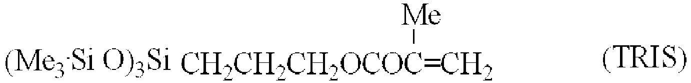 is widely used as a monomer for preparing soft contact lenses. A polymer obtained by copolymerizing TRIS with a hydrophilic monomer such as N,N-dimethylacrylamide or N-vinyl-2-pyrrolidone has advantages of transparency and high oxygen-permeability. However, a polysiloxane monomer having high hydrophobicity is not highly compatible with these hydrophilic monomers, so that phase separation and, consequently, turbidity occur, when a silicone hydrogel is produced from these to prepare an ophthalmic device. In addition, the siloxane bond is easily hydrolyzed. When the siloxane bond is brought into contact with a compound having an active hydrogen atom, such as water or alcohol, the siloxane component is gradually decomposed to cause the problem that the physical properties of the contact lens deteriorate during a long period of storing.

Patent Literature 1 describes a compound having a long alkyl chain on a silicon atom, such as 3-[tris(n-butyldimethylsiloxy)silyl]propyl methacrylate represented by the following formula, as a siloxane monomer having improved hydrolysis resistance.

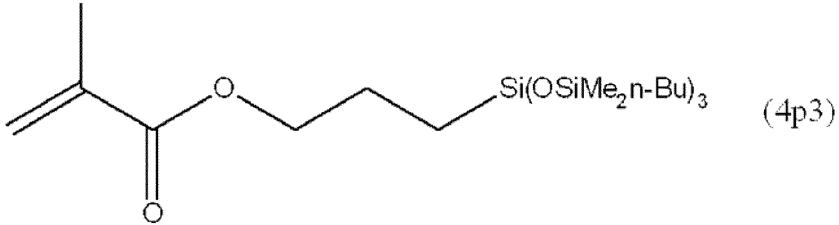

However, this type of compound in which an alkyl chain is bonded to a highly hydrophobic polysiloxane monomer does not have high compatibility with a hydrophilic monomer, so that phase separation and, consequently, turbidity occur when a silicone hydrogel is produced for preparing an ophthalmic device.

Patent Literature 2 describes a compound in which a polyether moiety is introduced as a linking group between a polysiloxane moiety and a polymerizable group. This compound having polyether structure has an improved compatibility with a hydrophilic monomer, but it is difficult to attain sufficient oxygen permeability due to a relatively decreased content of the polysiloxane structure.

### LIST OF THE PRIOR ART

### PATENT LITERATURE

Patent Literature 1: Japanese National Phase Publication No. 2011-503242
Patent Literature 2: Japanese Patent Application Laid-Open No. 2001-323024

### SUMMARY OF THE INVENTION

### PROBLEM TO BE SOLVED BY THE INVENTION

As described above, conventional polysiloxane monomers cannot provide high oxygen permeability, good compatibility with hydrophilic monomers, and high hydrolysis resistance, which are desired for making ophthalmic devices. Accordingly, there remains a need for a compound and a composition that overcome the aforesaid problems.

An object of the present invention is to provide a monomer having a polysiloxane structure and capable of providing a polymer having sufficient compatibility and hydrolysis resistance, while having high oxygen permeability useful for an ophthalmic device, and a method for producing the monomer.

### MEANS FOR SOLVING THE PROBLEMS

As a result of intensive studies to solve the above-mentioned problems, the present inventor has found that a compound having a bulky and hydrophilic substituent on a silicon atom of a polysiloxane provides a polymer having sufficient compatibility and hydrolysis resistance, while having beneficial oxygen permeability.

That is, the present invention provides a siloxane compound represented by the following formula (1): 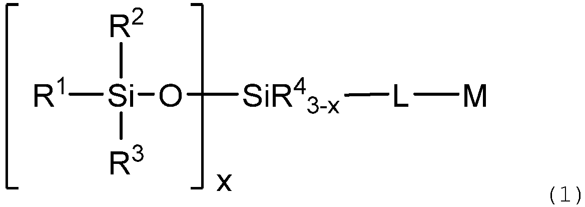 wherein M is a radical-polymerizable group, L is a divalent hydrocarbon group which has 2 to 20 carbon atoms and may contain one or more selected from an ether bond and a urethane bond, a part of the hydrogen atoms bonded to the carbon atoms may be substituted with a hydroxyl group, R¹ is, independently of each other, a group represented by formula (3-A) or formula (3-B), R² and R³ are, independently of each other, an alkyl group having 1 to 6 carbon atoms, R⁴ is a methyl group and x is an integer of from 1 to 3, 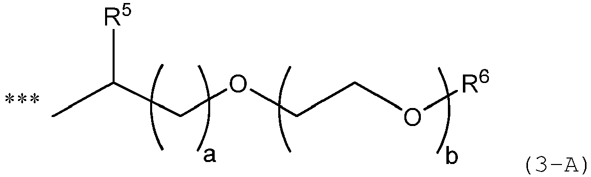 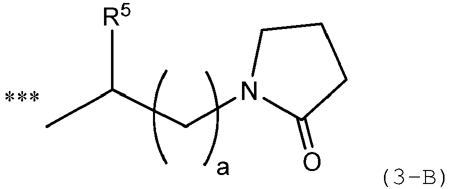 wherein R⁵ is a hydrogen atom or a methyl group, R⁶ is a hydrogen atom or an alkyl group having 1 to 4 carbon atoms, a is an integer of from 1 to 3, b is an integer of from 0 to 20, and the position represented by *** in the formulae bonds to a silicon atom in the formula (1).

### EFFECT OF THE INVENTION

The siloxane compound of the present invention provides a polymer having sufficient compatibility with other hydrophilic monomers, beneficial oxygen permeability, and hydrolysis resistance. Therefore, the siloxane compound of the present invention and the method for producing the compound are suitable for use in manufacturing an ophthalmic device.

### DETAILED DESCRIPTION OF THE INVENTION

The siloxane compound of the present invention is represented by the following formula (1): 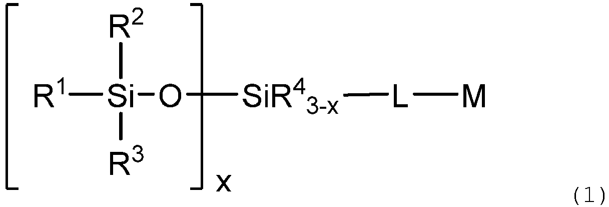 wherein M is a radical-polymerizable group, L is a divalent hydrocarbon group which has 2 to 20 carbon atoms and may contain one or more selected from an ether bond and a urethane bond, a part of the hydrogen atoms bonded to the carbon atoms may be substituted with a hydroxyl group, R¹ is, independently of each other, a group represented by formula (3-A) or formula (3-B), R² and R³ are, independently of each other, an alkyl group having 1 to 6 carbon atoms, R⁴ is a methyl group and x is an integer of from 1 to 3, 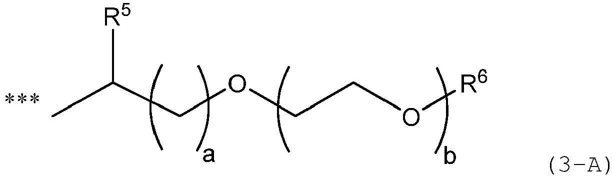 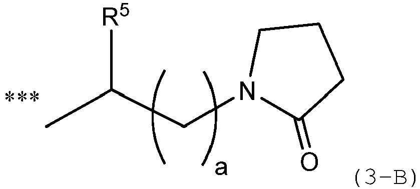 wherein R⁵ is a hydrogen atom or a methyl group, R⁶ is a hydrogen atom or an alkyl group having 1 to 4 carbon atoms, a is an integer of from 1 to 3, b is an integer of from 0 to 20, and the position represented by *** in the formulas bonds to a silicon atom in the formula (1).

The siloxane compound represented by the formula (1) is characterized in that a substituent bonded to a silicon atom is hydrophilic and has a bulky three-dimensional structure. The substituent imparts both compatibility and hydrolysis resistance at the siloxane site, so that a polymer derived from the siloxane compound has high oxygen permeability and stability.

In the formula (1), M is a radical-polymerizable group. Examples of the radical-polymerizable group include groups having acrylic or methacrylic groups, such as acryloyloxy, methacryloyloxy, acrylamide, and methacrylamide groups; N-vinylamide group, alkynyl groups, styryl group, indenyl group, alkenyl groups, cycloalkenyl groups, norborenyl group, and conjugated or unconjugated alkadiene groups. Among them, an acryloyloxy group, a methacryloyloxy group, an acrylamide group, a methacrylamide group, an N-vinylamide group, and a styryl group are preferable. From the viewpoint of ease of reaction, groups having an acrylic group or a methacrylic group are preferable. Particularly preferred are an acryloyloxy group, a methacryloyloxy group, an acrylamide group, and a methacrylamide group and, more preferably, an acryloyloxy group and a methacryloyloxy group.

In the formula (1), L is a divalent hydrocarbon group having from 2 to 20 carbon atoms, preferably from 2 to 10 carbon atoms, which may contain one or more selected from an ether bond and a urethane bond. In the divalent hydrocarbon group, a part of the hydrogen atoms bonded to the carbon atoms may be substituted with a hydroxyl group. Examples of the divalent hydrocarbon group include ethylene, propylene, 1-methylpropylene, 1,1-dimethylpropylene, 2-methylpropylene, 1,2-dimethylpropylene, 1,1,2-trimethylpropylene, butylene, 2-methyl-1,4-butylene, 2,2-dimethyl-1,4-butylene, 2,2-dimethyl-1,4-butylene, 2,3-dimethyl-1,4-butylene, 2,2,3-trimethyl-1,4-butylene, pentylene, hexanilene, heptanilene, octanylene, nonanylene and decanylene, groups composed of the aforesaid groups linked to each other via an ether or urethane bond, and the aforesaid groups wherein a part of the hydrogen atoms bonded to a carbon atom is substituted with a hydroxyl group. Examples of the group containing an ether bond include polyalkylene oxide such as polyethylene oxide, polypropylene oxide, and polyethylene-propylene oxide. Examples of the group containing the urethane bond include alkoxycarbonylaminoethyl esters such as ethoxycarbonylaminoethyl ester, propoxycarbonylaminoethyl ester, and butoxycarbonylaminoethyl ester. When L is a group containing a urethane bond, L is bonded to a silicon atom via a divalent alkyl, such as Si-(CH₂)_{y}-OCONH-(CH₂)ₓ-M.

L is preferably represented by the following formula (2-A) or formula (2-B). 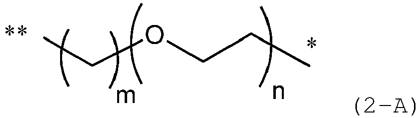 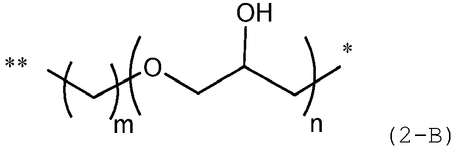 wherein m is 2 or 3, n is an integer of from 0 to 5, the position represented by ** bonds to a silicon atom of the formula (1), and the position represented by * bonds to M of the formula (1) .

L is preferably a group represented by the formula (2-A) or the formula (2-B) which has 2 to 9 carbon atoms, n is 0 to 2 and m is 2 to 3. Particularly preferably, L is a group having 2 to 7 carbon atoms represented by the above formula (2-A) or formula (2-B), wherein n is 0 to 1 and m is 2 to 3 carbon atoms.

In the formula (1), R¹ is, independently of each other, a group represented by the formula (3-A) or the formula (3-B). R² and R³ are, independently of each other, an alkyl group having 1 to 6 carbons, such as a methyl group, an ethyl group, a propyl group, a butyl group, a pentyl group, and a hexyl group, preferably a methyl group. R⁵ is a hydrogen atom or a methyl group. R⁶ is a hydrogen atom or an alkyl group having 1 to 4 carbon atoms, preferably an alkyl group, more preferably a methyl group, an ethyl group, or a propyl group. a is an integer of from 1 to 3, preferably 1 or 2. b is an integer of from 0 to 20, preferably from 0 to 12, more preferably 0 or from 1 to 5. If the number of the carbon atoms is too large, steric hindrance becomes larger and, further, the content of the siloxane component becomes smaller, so that the characteristics derived from the siloxane are lowered.

Preferably, in the above formula (3-A) and (3-B), R⁵ is a hydrogen atom or a methyl group, R⁶ is a methyl group, an ethyl group, or a propyl group, a is 1 or 2, b is an integer of from 0 to 12, and R² and R³ are, independently of each other, alkyl groups having 1 to 3 carbons.

Particularly preferably, in the above formula (3-A) and (3-B), R⁵ is a hydrogen atom, R⁶ is a methyl group, an ethyl group, or a propyl group, a is 1 or 2, b is an integer of from 0 to 5, and R² and R³ are methyl groups.

In the above formula (1), x is an integer of from 1 to 3, preferably 2 or 3.

Examples of the compound represented by the above formula (1) include the following structures, but the present invention is not limited by the following structures.

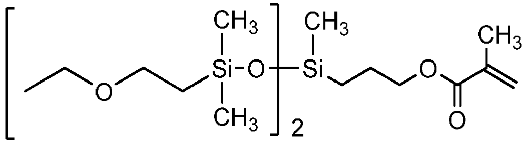

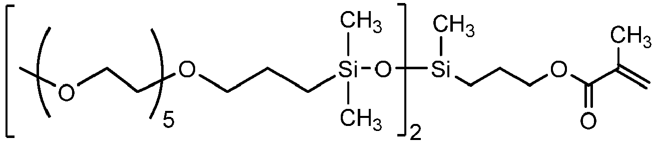

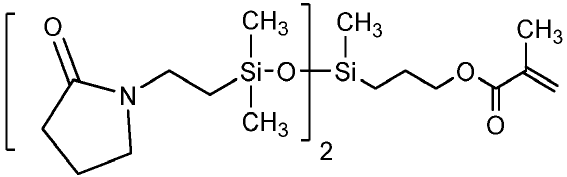

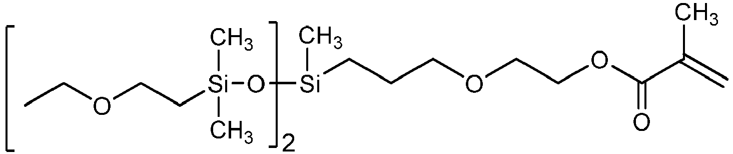

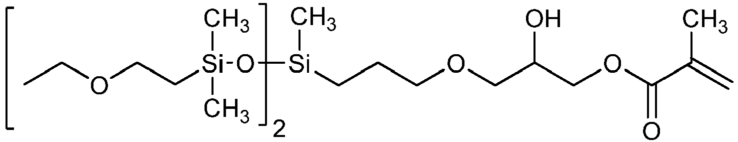

The siloxane compound of the present invention provides a polymer having excellent hydrolysis resistance. The hydrolysis resistance is evaluated by, for example, the ratio of the gas chromatography (GC) area % obtained by the GC measurement of the compound after being heated to the GC area % for the compound at the start of heating (0 hour) . Here, a 5% compound solution in water/2-propanol containing 5% by mass of acetic acid is heated at 80 degrees C for 168 hours. The siloxane compound of the present invention may have a GC area of 80% or more of the compound after being heated, relative to the GC area of the compound at the start of heating (0 hour).

The present invention further provides a method for producing the compound represented by the formula (1).

### (Preparing Method 1)

In a first preparing method, the compound represented by the above formula (1) is obtaind by reacting a siloxane compound represented by the following formula (4) 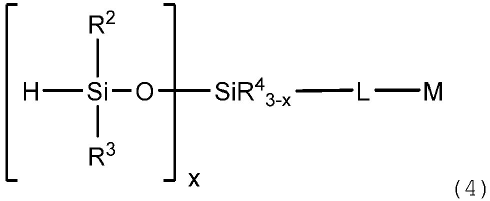 wherein M, L, R², R³, R⁴, and x are as described above,
with an unsaturated group-containing compound represented by the following formula (5-A) or (5-B), 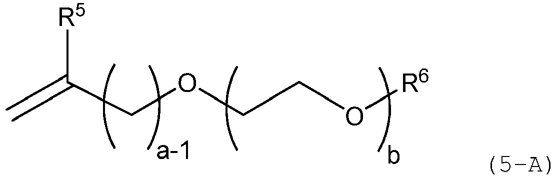 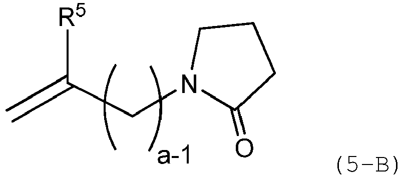 wherein R⁵, R⁶, a, and b are as described above.

The compound represented by the formula (4) is obtained by reacting an alkoxysilane compound represented by the following formula (6) 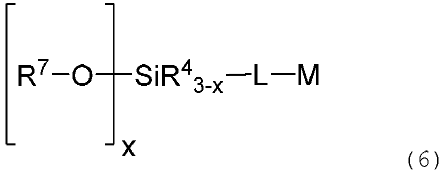 wherein M, L, R⁴ and x are as described above, and R⁷ is a monovalent hydrocarbon group having 1 to 5 carbon atoms, with a halogenated silane compound represented by the following formula (7) 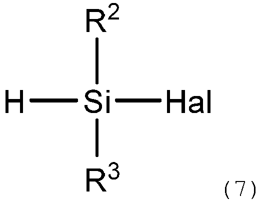 wherein R² and R³ are as described above, and Hal is a halogen atom.

R⁷ is a hydrocarbon group having 1 to 5 carbon atoms. Preferred are methyl, ethyl, propyl and butyl groups, particularly preferred are methyl and ethyl groups.

Hal is a halogen atom. The halogen atom is preferably a fluorine, a chlorine, a bromine or an iodine atom, particularly preferably a chlorine or a bromine atom and, even more preferably, a chlorine atom. The halogen atom is not particularly limited, but chlorine is preferable from the viewpoint of availability and reactivity.

The preparing method 1 may be conducted according to any conventional method. For example, 1 molar equivalent or more of the halogenated silane compound (7) and water are added to the alkoxysilane compound (6) and reacted to obtain the compound represented by the formula (4). For example, the reaction of 3-methacryloxypropyltrimethoxysilane with chlorodimethylsilane gives 3-methacryloxypropyltris(dimethylsiloxy)silane. Methanol and hydrogen chloride are by-producted at this time. Then, one or more molar equivalents of the unsaturated group-containing compound (5-A) or (5-B) are added to the obtained compound (4), and addition reacted to obtain a siloxane compound represented by the above formula (1). The addition reaction is carried out preferably in the presence of a hydrosilylation catalyst. These reactions are conducted typically at a temperature of from about 0 degrees C to about 150 degrees C. The reaction temperature is not particularly limited, but preferebly does not exceed the boiling point of the solvent used.

### (Preparing Method 2)

In a second preparing method, the compound represented by the above formula (1) is obtained by reacting a siloxane compound represented by the following formula (8) 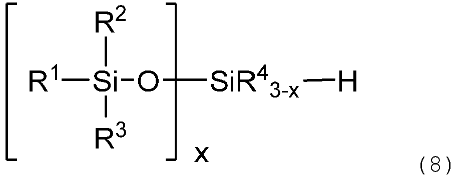 wherein R¹, R², R³, R⁴ and x are as described above,
with an unsaturated group-containing compound represented by the following formula (9).

**L'-M** (9)

In the formula (9), M is a radical polymerizable group as described above. For ease of reaction, a group having an acrylic group or a methacrylic group is preferable, particularly preferably an acryloyloxy group, a methacryloyloxy group, an acrylamide group, or a methacrylamide group and, more preferably, an acryloyloxy group or a methacryloyloxy group. L' is a monovalent hydrocarbon group having 2 to 20 carbon atoms, preferably 2 to 10 carbon atoms, having an unsaturated bond at its terminal. L' may contain one or more selected from an ether bond and a urethane bond, and a part of the hydrogen atoms bonded to a carbon atom may be substituted with a hydroxyl group.

L' is a monovalent hydrocarbon group which changes into the afore-mentioned divalent hydrocarbon group denoted by L and has a carbon-carbon unsaturated bond at the terminal opposite M. L' is preferably represented by the following formula (2-A') or formula (2-B'). 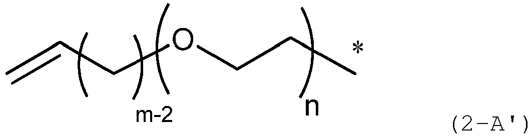 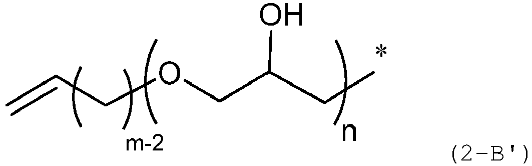 wherein m and n are as described above, and the position indicated by * in the formula bonds to M in the formula (9).

Examples of the unsaturated group-containing compound represented by the above formula (9) include allyl (meth)acrylate, 2-allyloxyethyl (meth)acrylate, 3-allyloxy-2-hydroxypropyl (meth)acrylate, and N-allylacrylamide, but are not limited to these.

The compound represented by formula (8) is obtained by reacting an alkoxysilane compound represented by formula (10) 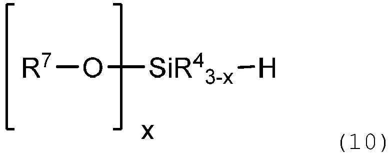 wherein R⁴, R⁷ and x are as described above,
with a halogenated silane compound represented by the following formula (11), 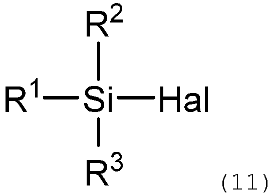 wherein R¹, R², R³ and Hal are as described above.

The preparing method 2 may be conducted according to any conventional method. For example, 1 molar equivalent or more of the halogenated silane compound (11) and water are added to the alkoxysilane compound (10) and reacted to obatin the compound represented by the formula (8). For example, the reaction of trimethoxysilane with chloro(2-ethoxyethyl)dimethylsilane gives tris(2-ethoxyethyldimethylsiloxy)silane. Methanol and hydrogen chloride are by-producted at this time. Then, 1 molar equivalent or more of the unsaturated group-containing compound (9) is added to the obtained compound represented by the formula (8), and addition-reacted to obtain a siloxane compound represented by the formula (1). The addition reaction is preferably carried out in the presence of a hydrosilylation catalyst. These reactions are typically conducted at a temperature of from about 0 degrees C to about 150 degrees C. The reaction temperature is not particularly limited, but preferably does not exceed the boiling point of the solvent.

In the above production methods 1 and 2, the amount of the halogenated silane compound (7) or (11) and the amount of water added are typically so as to give a ratio of 1 molar equivalent or more relative to a molar equivalent of the alkoxysilane compound (6) or (10). If it is less than the lower limit, the alkoxy group remains, which is not preferable. Although there is no upper limit in the above, the ratio is preferably 1 to 3. Too large amounts of the halogenated silane compound and water are not preferable in terms of yield and economy.

The amount of the unsaturated group-containing compound represented by the above formulas (5-A), (5-B) and (9) added may typically be so as to give a ratio of 1 molar equivalent or more relative to a molar equivalent of the compound (4) or (8). If it is less than the above value, the hydrosilyl group remains, which is not preferable. The upper limit is not limited, but the ratio is preferably 1 to 3 molar equivalents. Too large amounts of the compound having an unsaturated group are not preferable in terms of yield and economy.

The hydrosilylation catalyst may be any conventional one. For example, noble metal catalysts, in particular platinum catalysts derived from chloroplatinic acid, are suitable. In particular, it is preferable to completely neutralize the chloride ions of chloroplatinic acid with sodium bicarbonate to improve the stability of the platinum catalyst. The amount of the catalyst may be such as to cause the addition reaction to proceed. The temperature in the addition reaction is not particularly limited, and may be appropriately adjusted. Particularly, 20 to 150 degrees C, more preferably 50 to 120 degrees C is preferable. The reaction time is, for example, 1 to 12 hours, preferably 3 to 8 hours.

In the aforesaid reaction, a solvent is preferably used. The solvent is not particularly limited. For example, a hydrocarbon solvent such as hexane or heptane; an aromatic solvent such as toluene, an ether solvent such as tetrahydrofuran; a ketone solvent such as methyl ethyl ketone or N,N-dimethylformamide; an ester solvent such as ethyl acetate are suitably used.

After completion of the reaction, purification may be performed according to a conventional method. For example, the product is isolated by washing the organic layer with water and then removing the solvent. Alternatively, vacuum distillation, activated carbon treatment may be conducted.

An example of the preparing method 1 of the present invention will be described below. 1.2 molar equivalents of the halogenated silane compound (7) and water are simultaneously added dropwise to the alkoxysilane compound (6) . At this time, the dropping rate is adjusted so that the internal temperature is 30 degrees C or lower. After the dropwise addition, the reaction is completed after about 1 hour at room temperature. After completion of the reaction, the organic layer is washed with water until the pH of the washing liquid is near neutral (pH=6 to 8), and the organic layer is taken out. The siloxane compound represented by the above formula (4) is obtained by distilling off the solvent and any unreacted raw material present in the organic layer at a reduced pressure. Then, 1.0 molar equivalent of the unsaturated group-containing compound (5-A) or (5-B) is added to the siloxane compound (4), and heated to an internal temperature of 80 degrees C. A solution of a sodium bicarbonate-neutralized chloroplatinic acid-vinylsiloxane complex in toluene (platinum content: 0.5wt%) is added in an amount of 5ppm of platinum. The reaction is completed after about 3 hours at an internal temperature of 80 degrees C or more. Then, the organic layer is washed three times with a methanol-water solution (methanol: water=2:1) and is taken out. The siloxane compound represented by the above formula (1) is obtained by distilling off the solvent and any unreacted raw material present in the organic layer at a reduced pressure.

As an example of the preparing method 2 of the present invention, 1.2 molar equivalents of the halogenated silane compound (11) and water are simultaneously added dropwise to the alkoxysilane compound (10) . At this time, the dropping rate is adjusted so that the internal temperature is 30 degrees C or lower. After the dropwise addition, the reaction is completed after about 2 hours at room temperature. After completion of the reaction, the organic layer is washed with water until the pH of the washing liquid is near neutral (pH=6 to 8), and the organic layer is taken out. The siloxane compound represented by the above formula (8) is obtained by distilling off the solvent and any unreacted raw material present in the organic layer at a reduced pressure. Then, 1.0 molar equivalent of the unsaturated group-containing compound (9) is added to the siloxane compound (8), and heated to an internal temperature of 80 degrees C. A solution of a sodium bicarbonate-neutralized chloroplatinic acid-vinylsiloxane complex in toluene (platinum content: 0.5wt%) is added in an amount of 5ppm of platinum. The reaction is completed after about 3 hours at an internal temperature of 80 degrees C or more. Then, 100 parts by mass of n-hexane is added to the organic layer, the organic layer is washed three times with a methanol-water solution (methanol:water=3:1), and is taken out. The siloxane compound represented by the above formula (1) is obtained by distilling off the solvent and any unreacted raw material present in the organic layer at a under reduced pressure.

The siloxane compound of the present invention may provide polymers having the repeating units derived from the addition polymerization of radical polymerizable groups of the siloxane compound. The siloxane compound of the present invention has good compatibility with other compounds having a group polymerizing with the radical polymerizable group, M, of the siloxane compound, such as a (meth)acrylic group, hereinafter referred to as a polymerizable monomer or hydrophilic monomer. Therefore, a colorless transparent copolymer can be obtained by copolymerization with the polymerizable monomer. The present siloxan compound may polymerize with itself. As described above, the compound of the present invention provides a polymer having excellent hydrolysis resistance. In the preparation of a copolymer containing the repeating units derived from the siloxane compound of the present invention and another polymerizable (hydrophilic) monomer, the amount of the present siloxane compound is preferably 50 to 90 parts by mass, more preferably 60 to 80 parts by mass, relative to 100 parts by mass of the total of the present siloxane compound and the polymerizable (hydrophilic) monomer.

Examples of the polymerizable monomer include acrylic monomers such as (meth)acrylate, methyl (meth)acrylate, ethyl (meth)acrylate, (poly)ethylene glycol di(meth)acrylate, polyalkylene glycol mono(meth)acrylate, polyalkylene glycol monoalkylether(meth)acrylate, trifluoroethyl(meth)acrylate, and 2,3-dihydroxypropyl(meth)acrylate; acrylic acid derivatives such as N,N-dimethyl acrylamide, N,N-diethyl acrylamide, N-acryloylmorpholine, and N-methyl(meth)acrylamide; other unsaturated aliphatic or aromatic compounds such as crotonic acid, cinnamic acid, vinylbenzoic acid; and siloxane monomers having polymerizable groups such as a (meth) acrylic group. These may be used alone or in combination of two or more.

The copolymerization of the compound of the present invention and the other polymerizable monomer may be carried out according to any conventional method. For example, a known polymerization initiator such as a thermal polymerization initiator or a photopolymerization initiator may be used. Examples of the initiator include 2-hydroxy-2-methyl-1-phenyl-propane-1-one, azobisisobutyronitrile, azobisidimethylvaleronitrile, benzoyl peroxide, tert-butyl hydroperoxide, and cumene hydroperoxide. These polymerization initiators can be used alone or as a mixture of two or more kinds thereof. The amount of the polymerization initiator is preferably 0.001 to 2 parts by mass, preferably 0.01 to 1 part by mass, per 100 parts by total mass of the polymerization components.

The (co)polymer containing the repeating units derived from the compound of the present invention has excellent oxygen permeability and excellent hydrolysis resistance. Accordingly, the (co)polymer is suitable for manufacturing ophthalmic devices such as contact lenses, intraocular lenses, and artificial corneas. A method for manufacturing an ophthalmic device from the (co)polymer is not particularly limited, and may follow a conventional method of manufacturing an ophthalmic device. For example, a cutting method or a mold method may be used to attain the shape of a lens such as a contact lens and intraocular lens.

### EXAMPLES

The following Examples and Comparative examples are given to explain the present invention in more detail, but the present invention is not limited by the following Examples.

The analytical methods performed in the following Examples and Comparative Examples are described below.

### a: Nuclear Magnetic Resonance Analysis (NMR)

The structures of the siloxane compounds were confirmed by ¹H-NMR spectrometry. ECS500 provided by JEOL was used, and deuterochloroform was used as a measurement solvent.

### b. Gas Chromatographic Analysis (GC)

### i. Device and Parameters

Device: GC system 7890A type, ex Agilent. Detector: Flame ionization detector (FID). Column:J & W HP-5 MS(0.25 mm^{∗}30 m^{∗}0.25µm) Carrier gas: Helium, constant flow rate: 1.0 mL/min. Injected sample volume: 1.0µL. Split ratio :50:1 Injection port temperature :250 degrees C. Detector temperature :300 degrees C.

### ii. Temperature program

Start temperature: 50 degrees C. Start time: 2 min. Gradient: 10 degrees C./min; End temperature: 300 degrees C. (retention time: 10 min).

### iii. Sample preparation

A sample solution was placed directly in a GC vial without being diluted.

### [Synthesis Example 1]

To a 1L four-neck eggplant flask equipped with a dimroth, a thermometer, and two dropping funnels, 400.0g of 3-methacryloxypropylmethyldimethoxysilane was added, and 320.0g of chlorodimethylsilane and 80.0g of deionized water were added dropwise from the dropping funnels, while keeping the internal temperature at or below 30 degrees C. After completion of the dropwise addition, the reaction liquid was stirred at room temperature for 1 hour, and disappearance of the starting materials was confirmed by gas chromatography (GC) and the reaction step was ended. Then, the organic layer was transferred to a separatory funnel and washed three times with tap water. The organic layer was separated, the solvent and any unreacted starting materials were distilled off at an internal temperature of 50 degrees C and reduced pressure. The remaining was subjected to distillation under reduced pressure to obtain a siloxane compound represented by the following formula (12) . The yield was 530.2g.

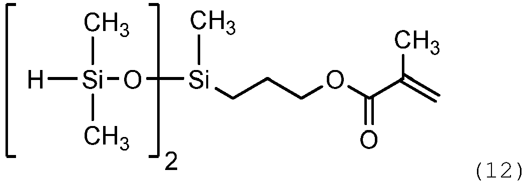

### [Synthesis Example 2]

Synthesis Example 1 was repeated, except that 3-methacryloxypropyltrimethoxysilane was used instead of 3-methacryloxypropylmethyldimethoxysilane to obtain a siloxane compound represented by the following formula (13). The yield was 558.0g.

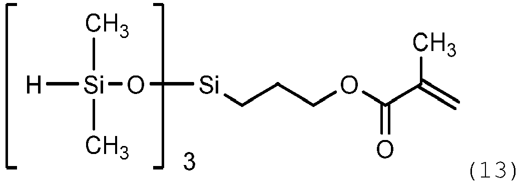

### [Example 1]

To a 200-mL three-neck eggplant flask equipped with a dimroth, a thermometer, and a dropping funnel, 35.0g of ethyl vinyl ether, 35.0g of toluene, 0.04g of a solution of sodium bicarbonate-neutralized chloroplatinic acid-vinylsiloxane complex in toluene (platinum content: 0.5wt%) were added, and 50.0g of the siloxane compound (12) obtained in Synthesis Example 1 was added dropwise from the dropping funnels. After completion of the dropwise addition, the reaction liquid was stirred at 40 degrees C for 5 hours, and disappearance of the starting materials was confirmed by gas chromatography (GC) and the reaction step was ended. Then, the organic layer was transferred to a separatory funnel and washed three times with a methanol-water solution (methanol:water=2:1). The organic layer was separated, and the solvent and any unreacted starting materials were distilled off at an internal temperature of 50 degrees C and reduced pressure to obtain a siloxane compound represented by the following formula (14) . The yield was 65.3g. The ¹H-NMR date are as shown below.
0.0ppm(15 H), 0.5ppm(2 H), 0.7ppm(4 H), 1.1ppm(6 H), 1.6ppm (2 H), 2.0ppm(3 H), 3.5-3.6ppm(8 H), 4.2ppm(2 H), 5.6ppm(1 H), 6.1ppm(1 H)

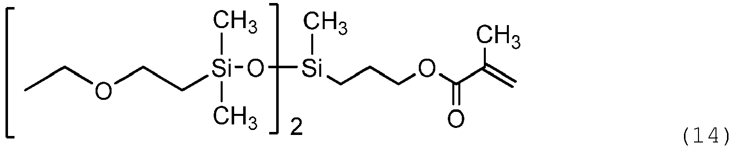

### [Example 2]

Example 1 was repeated, except that isopropyl vinyl ether was used instead of ethyl vinyl ether to obtain a siloxane compound represented by the following formula (15) . The yield was 66.0g. The ¹H-NMR date are as shown below.
0.0ppm(15 H), 0.5ppm(2 H), 0.6ppm(4 H), 1.1ppm(12 H), 1.6ppm (2 H), 2.0ppm(3 H), 3.5ppm(6 H), 4.2ppm(2 H), 5.6ppm(1 H), 6.1ppm(1 H)

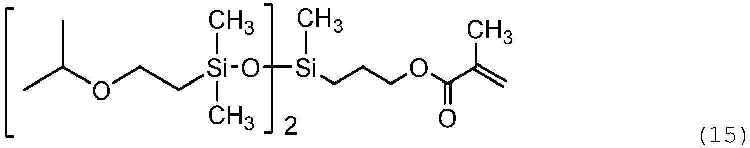

### [Example 3]

Example 1 was repeated, except that 3-methoxyethyl allyl ether was used instead of ethyl vinyl ether to obtain a siloxane compound represented by the following formula (17). The yield was 65.8g. The ¹H-NMR date are as shown below.
0.0ppm(15 H), 0.5ppm(6 H), 1.6ppm(6 H), 2.0ppm(3 H), 3.4-3.6ppm(18 H), 4.2ppm(2 H), 5.6ppm(1 H), 6.1ppm(1 H)

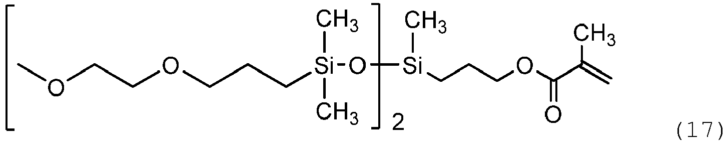

### [Example 4]

Example 1 was repeated, except that a methoxy-terminated allyl polyether having an average molecular weight of 240 was used instead of ethyl vinyl ether to obtain a siloxane compound represented by the following formula (18) . The yield was 72.5g. The ¹H-NMR date are as shown below.
0.0ppm(15 H), 0.5ppm(6 H), 1.6ppm(6 H), 2.0ppm(3 H), 3.4-3.6ppm(50 H), 4.2ppm(2 H), 5.6ppm(1 H), 6.1ppm(1 H)

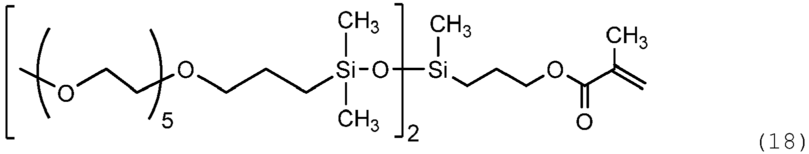

### [Example 5]

Example 1 was repeated, except that a methoxy-terminated allyl polyether having an average molecular weight of 550 was used instead of ethyl vinyl ether to obtain a siloxane compound represented by the following formula (19) . The yield was 75.3g. The ¹H-NMR is shown below.
0.0ppm(15 H), 0.5ppm(6 H), 1.6ppm(6 H), 2.0ppm(3 H), 3.4-3.6ppm(106 H), 4.2ppm(2 H), 5.6ppm(1 H), 6.1ppm(1 H)

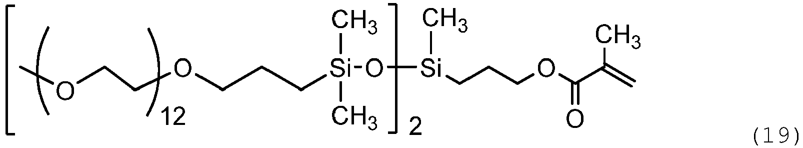

### [Example 6]

Example 1 was repeated, except that the ethyl vinyl ether was changed to N-vinyl pyrrolidone to obtain a siloxane compound represented by the following formula (20) . The yield was 54.6g. The ¹H-NMR date are as shown below.
0.0ppm(15 H), 0.5ppm(2 H), 1.1ppm(4 H), 1.6ppm(2 H), 2.0ppm (3 H), 2.1-2.3ppm(8 H), 3.3-3.4ppm(8 H), 4.2ppm(2 H), 5.6ppm(1 H), 6.1ppm(1 H)

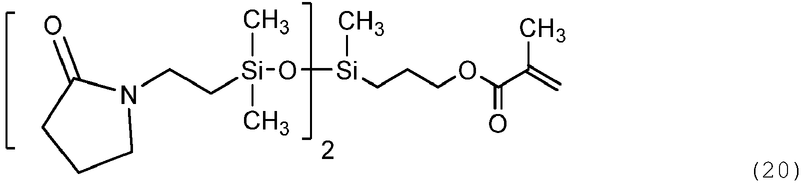

### [Example 7]

Example 1 was repeated, except that siloxane compound (13) obtained in Synthesis Example 2 was used instead of siloxane compound (12) to obtain a siloxane compound represented by the following formula (21). The yield was 70.4g. The ¹H-NMR date are as shown below.
0.0ppm(12 H), 0.5ppm(2 H), 0.7ppm(6 H), 1.1ppm(9 H), 1.6ppm (2 H), 2.0ppm(3 H), 3.5-3.6ppm(12 H), 4.2ppm(2 H), 5.6ppm (1 H),6.1ppm(1 H)

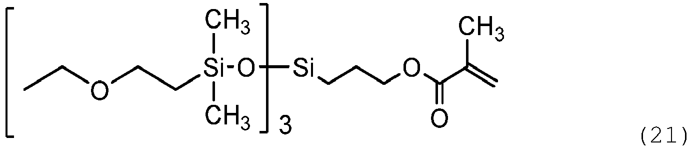

### [Synthesis Example 3]

To a 1L four-neck eggplant flask equipped with a dimroth, a thermometer, and two dropping funnels, 100.0 g of methyldimethoxysilane was added, and 700.0g of chloro(2-ethoxyethyl)dimethylsilane and 100.0g of deionized water were added dropwise from the dropping funnels, while keeping the internal temperature at or below 30 degrees C. After completion of the dropwise addition, the reaction liquid was stirred at room temperature for 1 hour, and disappearance of the starting materials was confirmed by gas chromatography (GC) and the reaction step was ended. Then, the organic layer was transferred to a separatory funnel and washed twice with tap water. The organic layer was separated, the solvent and any unreacted starting materials were distilled off at an internal temperature of 50 degrees C and reduced pressure. The remaining was subjected to distillation under reduced pressure to obtain a siloxane compound represented by the following formula (22) . The yield was 460.6g.

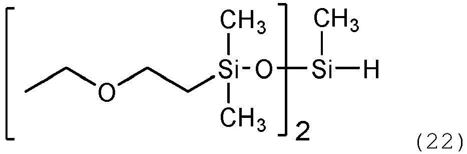

### [Synthesis Example 4]

Synthesis Example 1 was repeated, except that chloro(3-(2-methoxyethoxy)propyl)dimethylsilane was used instead of chloro(2-ethoxyethyl)dimethylsilane to obtain a siloxane compound represented by the following formula (23). The yield was 453.0g.

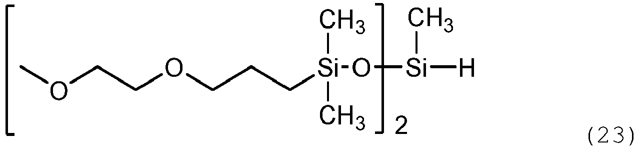

### [Synthesis Example 5]

Synthesis Example 1 was repeated, except that trimethoxysilane was used instead of methyldimethoxysilane to obtain a siloxane compound represented by the following formula (24). The yield was 487.1g.

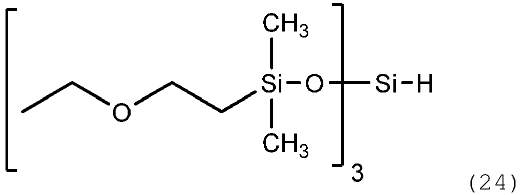

### [Example 8]

To a 200-mL three-neck eggplant flask equipped with a dimroth, a thermometer, and a dropping funnel, 50.0g of 2-allyloxyethyl methacrylate, 50.0g of toluene, and 0.04g of a solution of sodium bicarbonate-neutralized chloroplatinic acid-vinylsiloxane complex in toluene (platinum content 0.5wt%) were added, and 50.0g of the siloxane compound (22) obtained in Synthesis Example 3 was added dropwise from the dropping funnels. After completion of the dropwise addition, the reaction liquid was stirred at 50 degrees C for 5 hours, and disappearance of the starting materials was confirmed by gas chromatography (GC) and the reaction step was ended. Then, 100.0g of n-hexane was added to the organic layer, and the organic layer was transferred to a separatory funnel and washed three times with a methanol-water solution (methanol: water=3:1). The organic layer was separated, and the solvent and any unreacted starting materials were distilled off at an internal temperature of 50 degrees C and reduced pressure to obtain a siloxane compound represented by the following formula (25) . The yield was 89.5g. The ¹H-NMR date are as shown below.
0.0ppm(15 H), 0.5ppm(2 H), 0.7ppm(4 H), 1.1ppm(6 H), 1.6ppm (2 H), 2.0ppm(3 H), 3.4ppm(2 H), 3.5-3.6ppm(10 H), 4.2ppm(2 H), 5.6ppm(1 H), 6.1ppm(1 H)

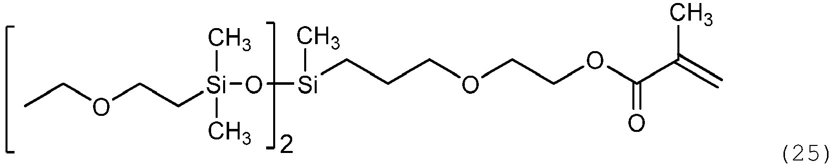

### [Example 9]

Example 8 was repeated, except that 3-allyloxy-2-hydroxypropyl methacrylate was used instead of 2-allyloxyethyl methacrylate to obtain the siloxane compound represented by the following formula (26) . The yield was 60.6g. The ¹H-NMR date are as shown below.
0.0ppm(15 H), 0.5ppm(2 H), 0.7ppm(4 H), 1.1ppm(6 H), 1.6ppm (2 H), 2.0ppm(3 H), 3.4-3.6ppm(12 H), 4.0ppm(1 H), 4.2ppm(2 H), 5.6ppm(1 H), 6.1ppm(1 H)

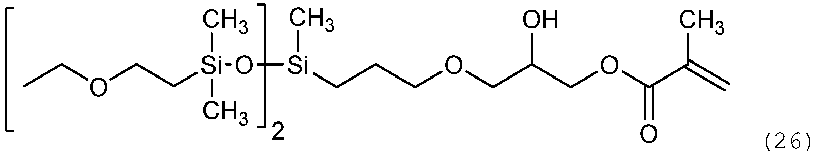

### [Example 10]

Example 8 was repeated, except that N-allylacrylamide was used instead of 2-allyloxyethyl methacrylate to obtain a siloxane compound represented by the following formula (27). The yield was 50.2g. The ¹H-NMR date are as shown below.
0.0ppm(15 H), 0.6ppm(2 H), 0.7ppm(4 H), 1.1ppm(6 H), 1.6ppm (2 H), 2.0ppm(3 H), 3.3ppm(2 H), 3.5-3.6ppm(8 H), 5.6ppm(1 H), 5.7ppm(1 H), 6.1ppm(1 H), 6.3ppm(1 H)

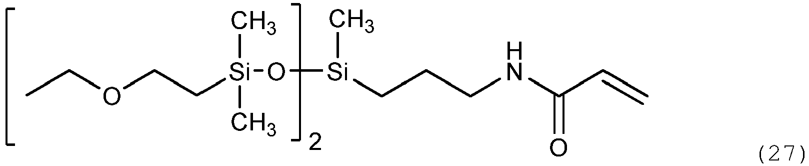

### [Example 11]

Example 8 was repeated, except that the siloxane compound (23) obtained in Synthesis Example 4 was used instead of the siloxane compound (22) to obtain a siloxane compound represented by the following formula (28). The yield was 53.2g. The ¹H-NMR date are as shown below.
0.0ppm(15 H), 0.5ppm(6 H), 1.6ppm(6 H), 2.0ppm(3 H), 3.4-3.7ppm(22 H), 4.2ppm(2 H), 5.6ppm(1 H), 6.1ppm(1 H)

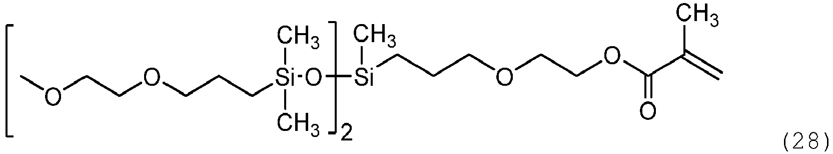

### [Example 12]

Example 9 was repeated, except that the siloxane compound (24) obtained in Synthesis Example 5 was repeated instead of the siloxane compound (22) to obtain a siloxane compound represented by the following formula (29). The yield was 58.3g. The ¹H-NMR date are as shown below.
0.0ppm(12 H), 0.5ppm(2 H), 0.7ppm(6 H), 1.1ppm(9 H), 1.6ppm (2 H), 2.0ppm(3 H), 3.4-3.6ppm(16 H), 4.0ppm(1 H), 4.2ppm(2 H), 5.6ppm(1 H), 6.1ppm(1 H)

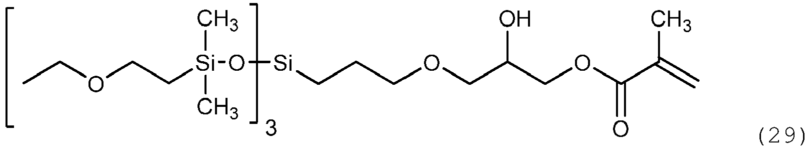

### [Comparative Example 1]

Example 7 was repeated, except that 1-pentene was used instead of ethyl vinyl ether to obtain a siloxane compound represented by the following formula (30). The yield was 73.5g.

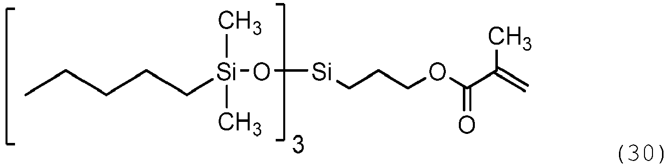

### [Comparative Example 2]

In Comparative Example 2, a compound represented by the following formula, which is widely used as a monomer for soft contact lenses, was used.

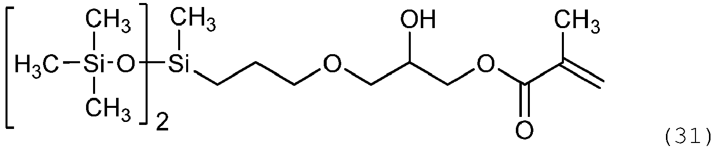

### [Hydrolysis Resistance Test]

The following test was conducted on the siloxane compounds obtained above.

To a 20-mL screw-capped vial, 0.1g of the siloxane compound, 3.90g of 2-propanol, 0.24g of acetic acid, 0.90g of distilled water, and 2mg of 2,6-di-t-butyl-4-methylphenol as a polymerization inhibitor were placed and well mixed, and the screw-capped vial was sealed. The screw-capped vial was left at 80 degrees C for 168 hours. The mixed solution immediately after being mixed (0 hour) and the mixed solution after being left for 168 hours were subjected to gas chromatography, respectively. The conditions of the gas chromatography are as described above.

Each peak area obtained by gas chromatography is proportional to the amount of each component contained.

The percentage (%) of the peak area of the siloxane compound after 168 hours relative to the peak area (100%) of the siloxane compound immediately after being mixed (0 hour) was calculated. The result is as shown in Table 1. The higher the numerical value, the smaller the weight decrease due to hydrolysis, which means that the hydrolysis resistance is higher.

[Method for Evaluating Compatibility with a Hydrophilic Monomer]

Compatibility with a hydrophilic monomer, N-vinylpyrrolidone (NVP), which is widely used in the manufacture of ophthalmic devices, was evaluated. The siloxane compound and NVP were mixed in equal weights and stirred for 10 minutes at 25 degrees C. After stirring, the mixture was allowed to stand at 25 degrees C for 5 hours, and the appearance thereof was visually observed to evaluate on the basis of the following indices.
G: Uniform and clear, and B: Turbid
The results are as shown in Table 1.

**Table 1**

| | Siloxane Compound No. | Hydrolysis resistance: percentage (%) of the GC area after 168 hours | Compatibility |
|---|---|---|---|
| EXAMPLE 1 | (14) | 88.9 | G |
| EXAMPLE 2 | (15) | 91.5 | G |
| EXAMPLE 3 | (17) | 89.7 | G |
| EXAMPLE 4 | (18) | 87.3 | G |
| EXAMPLE 5 | (19) | Decomposition product, not detected ¹⁾ | G |
| EXAMPLE 6 | (20) | 82.4 | G |
| EXAMPLE 7 | (21) | 92.3 | G |
| EXAMPLE 8 | (25) | 90.1 | G |
| EXAMPLE 9 | (26) | 83.0 | G |
| EXAMPLE 10 | (27) | 81.2 | G |
| EXAMPLE 11 | (28) | 82.5 | G |
| EXAMPLE 12 | (29) | 84.2 | G |
| Comparative Example 1 | (30) | 89.6 | B |
| Comparative Example 2 | (31) | 19.0 | G |

Note 1): The siloxane compound of Example 5 could not be analyzed by GC because of its large carbon number and large molecular weight. However, the hydrolysis resistance of the siloxane compound of Example 5 was good, because no low molecular weight decomposition product from the siloxane compound due to hydrolysis was detected.

As shown in Table 1, the siloxane compound of Comparative Example 1 was not compatible with NVP to cause white turbidity. The siloxane compound of Comparative Example 2 was hydrolyzed under the above-mentioned conditions at 80 degrees C for 168 hours, and the content of the component was remarkably reduced. That is, the hydrolysis resistance is inferior. On the other hand, the siloxane compounds of the present invention have excellent compatibility with NVP, which is hydrophilic, show only a small decrease in the peak area ratio under the above conditions, do not give a decomposition product, and are excellent in hydrolysis resistance. Furthermore, the present siloxane compounds have a polysiloxane structure and, therefore, can be copolymerized with other polymerizable monomers to provide a cured product which is colorless and transparent and has excellent oxygen permeability.

### INDUSTRIAL APPLICABILITY

The siloxane compound of the present invention is excellent in compatibility with other polymerizable monomers such as (meth)acrylic monomers and provides a polymer by being (co)polymerized, which copolymer has excellent hydrolysis resistance and is suitable for application to medical devices such as ophthalmic devices. The compound and the preparation method of the present invention are useful for the manufacture of ophthalmic devices, such as contact lens materials, intraocular lens materials, and artificial corneal materials.

## Claims

1. A compound represented by the following formula (1): 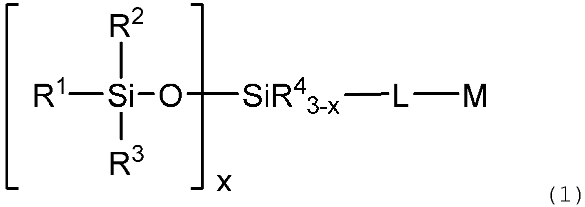 wherein M is a radical-polymerizable group, L is a divalent hydrocarbon group which has 2 to 20 carbon atoms and may contain one or more selected from an ether bond and a urethane bond, a part of the hydrogen atoms bonded to the carbon atoms may be substituted with a hydroxyl group, R¹ is, independently of each other, a group represented by formula (3-A) or formula (3-B), R² and R³ are, independently of each other, an alkyl group having 1 to 6 carbon atoms, R⁴ is a methyl group and x is an integer of from 1 to 3, 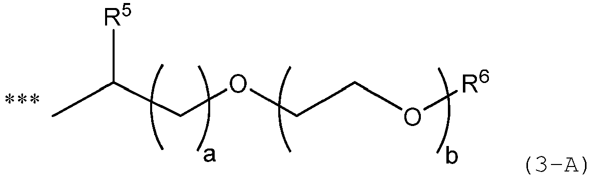 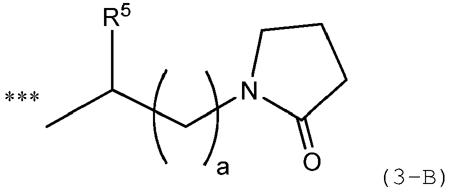 wherein R⁵ is a hydrogen atom or a methyl group, R⁶ is a hydrogen atom or an alkyl group having 1 to 4 carbon atoms, a is an integer of from 1 to 3, b is an integer of from 0 to 20, and the position represented by *** in the formulae bonds to a silicon atom in the formula (1).

2. The compound according to claim 1, wherein L is represented by the following formula (2-A) or (2-B) 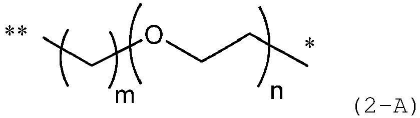 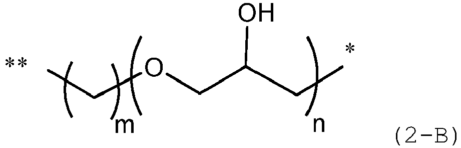 wherein m is 2 or 3, n is an integer of from 0 to 5, the position represented by ** bonds to a silicon atom of the formula (1), and the position represented by * bonds to M of the formula (1) .

3. The compound according to claim 2, wherein n is 0 or 1.

4. The compound according to claim 3, wherein m is 3 and n is 1.

5. The compound according to claim 3, wherein m is 3 and n is 0.

6. The compound according to any one of claims 1 to 5, wherein a is 1 or 2, b is an integer of from 0 to 12, and R⁶ is an alkyl group having 1 to 3 carbon atoms.

7. The compound according to any one of claims 1 to 5, wherein a is 1 or 2, b is an integer of from 0 to 5, R⁵ is a hydrogen atom, and R⁶ is an alkyl group having 1 to 3 carbon atoms.

8. The compound according to any one of claims 1 to 7, wherein R² and R³ are each a methyl group.

9. The compound according to any one of claims 1 to 8, wherein M is a radical having an acryloyl or methacryloyl group.

10. The compound according to claim 9, wherein M is an acryloyloxy group, a methacryloyloxy group, an acrylamide group or a methacrylamide group.

11. The compound according to any one of claims 1 to 10, wherein x is 2 or 3.

12. A polymer comprising repeating units derived from addition polymerization of the radical-polymerizable group, M, in the compound according to any one of claims 1 to 11.

13. A copolymer comprising repeating units derived from copolymerization of the radical-polymerizable group, M, of the compound according to any one of claims 1 to 11 with another compound having a group which is polymerizable with said radical-polymerizable group, M.

14. An ophthalmic device composed of the copolymer according to claim 13.

15. A method for preparing a compound represented by the following formula (1): 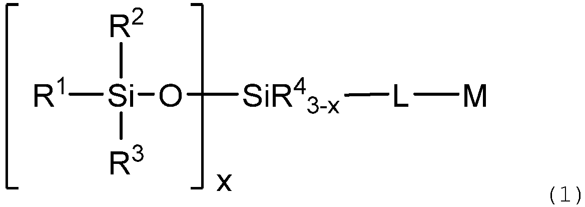 wherein M is a radical-polymerizable group, L is a divalent hydrocarbon group which has 2 to 20 carbon atoms and may contain one or more selected from an ether bond and a urethane bond, a part of the hydrogen atoms bonded to the carbon atoms may be substituted with a hydroxyl group, R¹ is, independently of each other, a group represented by formula (3-A) or formula (3-B), R² and R³ are, independently of each other, an alkyl group having 1 to 6 carbon atoms, R⁴ is a methyl group and x is an integer of from 1 to 3, 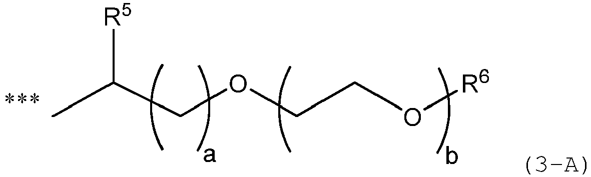 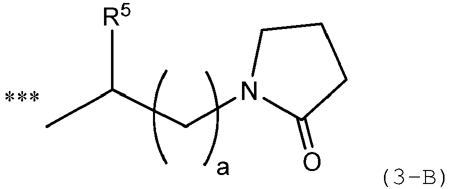 wherein R⁵ is a hydrogen atom or a methyl group, R⁶ is a hydrogen atom or an alkyl group having 1 to 4 carbon atoms, a is an integer of from 1 to 3, b is an integer of from 0 to 20, and the position represented by *** in the formulae bonds to a silicon atom of the formula (1),
the method comprising a step of reacting a siloxane compound represented by the following formula (4) 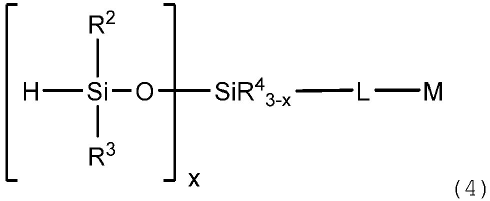 wherein M, L, R², R³, R⁴ and x are as defined above,
with an unsaturated group-containing compound represented by the following formula (5-A) or (5-B) 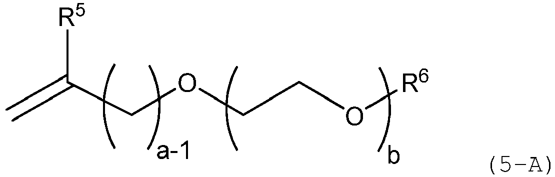 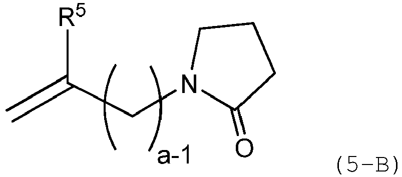 wherein R⁵, R⁶, a and b are as defined above,
to obtain the compound represented by the formula (1).

16. A method for preparing a compound represented by the following formula (1): 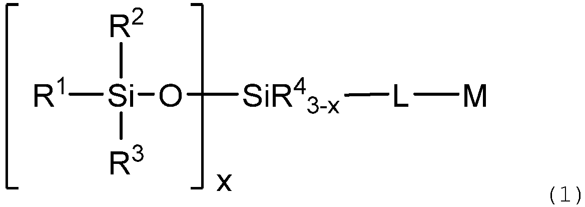 wherein M is a radical-polymerizable group, L is a divalent hydrocarbon group which has 2 to 20 carbon atoms and may contain one or more selected from an ether bond and a urethane bond, a part of the hydrogen atoms bonded to the carbon atoms may be substituted with a hydroxyl group, R¹ is, independently of each other, a group represented by formula (3-A) or formula (3-B), R² and R³ are, independently of each other, an alkyl group having 1 to 6 carbon atoms, R⁴ is a methyl group and x is an integer of from 1 to 3, 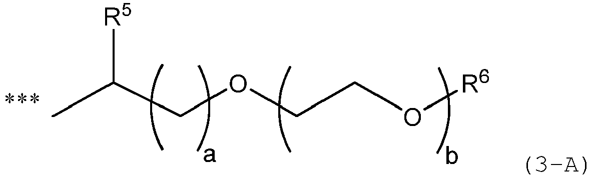 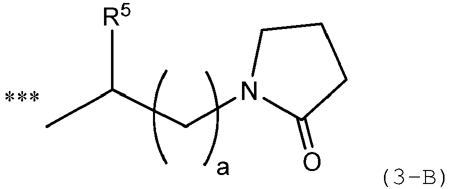 wherein R⁵ is a hydrogen atom or a methyl group, R⁶ is a hydrogen atom or an alkyl group having 1 to 4 carbon atoms, a is an integer of from 1 to 3, b is an integer of from 0 to 20, and the position represented by *** in the formulae bonds to a silicon atom in the formula (1),
the method comprising a step of reacting a siloxane compound represented by the following formula (8) 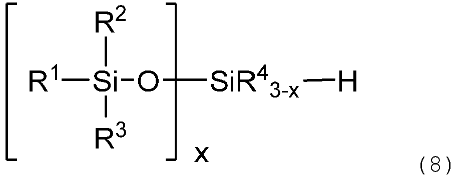 wherein, R¹, R², R³, R⁴ and x are as defined above,
with an unsaturated group-containing compound represented by the following formula (9)
**L'-M** (9)
wherein M is a radical-polymerizable group, L' is a monovalent hydrocarbon group which has 2 to 20 carbon atoms, has an unsaturated bond at its terminal and may contain one or more selected from an ether bond and a urethane bond, and a part of the hydrogen atoms bonded to the carbon atoms may be substituted with a hydroxyl group,
to obtain the compound represented by the formula (1).

17. The method according to claim 16, wherein L' is represented by the following formula (2-A') or (2-B') 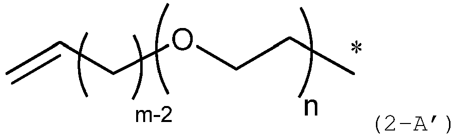 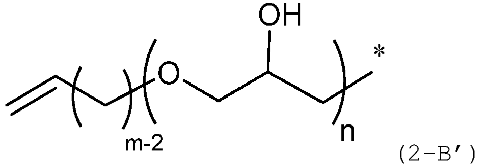 wherein m is 2 or 3, n is an integer of from 0 to 5, and the position represented by * bonds to M in the formula (9).

18. The method according to claim 17, wherein n is 0 or 1.

19. The method according to any one of claims 15 to 18, wherein a is 1 or 2, b is an integer of from 0 to 12, R⁶ is an alkyl group having 1 to 3 carbon atoms.

20. The method according to any one of claims 15 to 19, wherein R² and R³ are each a methyl group.

21. The method according to any one of claims 15 to 20, wherein M is a radical having an acryloyl or methacryloyl group.

22. The method according to claim 21, wherein M is an acryloyloxy group, a methacryloyloxy group, an acrylamide group or a methacrylamide group.

23. The method according to any one of claims 15 to 22, wherein x is 2 or 3.
